# EUROPEAN PATENT APPLICATION

(11) **EP 4 755 592 A1**
(43) Date of publication of application: **10.06.2026**
(21) Application number: 25170381.5
(22) Date of filing: 14.04.2025
(51) Int. Cl.: B25J 9/16, G01J 3/46, G05B 19/418, G05B 13/02, B01F 35/22, B01F 35/82

(54) **ARTIFICIAL INTELLIGENCE (AI) BASED BATCH TINTING ASSISTANT**

(30) Priority: 09.12.2024 IN 202411097083
(71) Applicant: Schneider Electric Systems USA, Inc., Foxborough, MA 02035 (US)
(72) Inventor: PATIL, Ashish, 411045 Maharashtra (IN); BHATTACHARYYA, Amitabha, 500019 Telangana (IN); SINHA, Bhaskar, 500019 Telangana (IN); SUBRAMANIAN, Mohan Raj, 630303 Tamil Nadu (IN); POLENI, Ravi, 500092 Telangana (IN); CHANDANAPURKAR, Prashant, 411045 Maharashtra (IN); PARMAR, Prashant, 411028 Maharashtra (IN)
(74) Representative: Friese Goeden Patentanwälte PartGmbB

(57) **Abstract**

A method of tinting liquids in an industrial mixing system. A color measurement unit receives a liquid batch sample and measures the sample to generate a color measurement value. A color digitization processor receives the color measurement value and executes an artificial intelligence (AI) tinting prediction engine. The Al tinting prediction engine stores the color measurement value in a database, which contains a plurality of historical batch sample color information, a plurality of target standard sample color measurement values, and a plurality of colorant information. The Al tinting prediction engine then defines delta errors between the color measurement value and a target standard sample measurement value. The Al tinting prediction engine then generates predicted colorants and colorant masses based on the delta errors and information stored within the database. The predicted colorants are then transmitted to the mixing system and mixed into the liquid batch.

## Description

### CROSS-REFERENCE TO RELATED APPLICATION

This application claims the benefit of Indian Patent Application No. 202411097083, filed December 9, 2024.

### BACKGROUND

Industrial tinting is the process of adjusting the color of a liquid product to match a specific target for its intended use. The conventional tinting process is lengthy, requiring multiple iterations of testing samples and adding colorants to achieve the desired color. In traditional paint tinting, a sample from a liquid batch is applied to a test panel, which is then cured to reveal the actual color. This test panel is compared to a reference panel of the target color to assess the match. A tinting expert from quality control evaluates the sample and target panels' colors to determine the appropriate colorants and their quantities to be added to the liquid batch to achieve the target color within a specified tolerance. Consequently, this process often results in significant time consumption and waste, as new test panels must be created for each evaluation.

The tinting process relies heavily on the expertise of a tinting specialist to achieve the desired results. A thorough understanding of product colors, colorants, and their respective strengths is essential for successful tinting. However, plant managers and operators often lack insight into the tinting process, including the duration of operations and the number of iterations required. This dependence on individual expertise can lead to variability in tinting outcomes, with results differing from expert to expert. Consequently, the inconsistency in the tinting process can affect batch-to-batch uniformity, making it challenging for plant managers and operators to effectively plan batch schedules.

Current conventional tools for measuring liquid color have limitations which prevent accurate measurement of color. Although spectrophotometers can determine color measurements, measuring liquids such as paints in existing systems introduce several potential sources of error. For instance, the meniscus of the liquid can affect the measurement accuracy, and freshly produced high viscous liquids such as paint often contain air bubbles that further compromise the precision of the readings.

### SUMMARY

Aspects of the present disclosure provide an artificial intelligence (AI) based liquid tinting solution incorporating system for accurate measurement, historical tinting information, and colorant dosing estimation to provide efficient tinting process within an industrial mixing system.

In an aspect, a method of tinting liquids in an industrial mixing system comprises collecting a liquid batch sample from a liquid batch in an industrial mixing system and measuring the liquid batch sample with a sensor to generate a batch color measurement value associated with the liquid batch sample. The method further comprises transmitting the batch color measurement value to a color digitization processor and executing, by the color digitization processor, an Al tinting prediction engine. Executing the Al tinting prediction engine comprises storing the batch color measurement value in a color digitization database wherein the database comprises a plurality of historical batch sample color information, a plurality of target standard sample color measurement values, and a plurality of colorant information wherein the colorant information comprises a colorant value. Executing the Al tinting prediction engine further includes defining a delta error between the batch color measurement value and a target standard sample color measurement value. Executing the Al tinting prediction engine also includes modeling tinting of the liquid batch by at least one predicted colorant of the plurality of colorant information and a mass of the colorant to achieve the target standard sample color measurement by weighting the delta error, the batch color measurement value, a batch volume, the plurality of historical batch sample color information, and the plurality of colorant information. The method further comprises receiving, by the industrial mixing system, the predicted colorant and the mass of the colorant and mixing, by the industrial mixing system, one or more colorants based on the predicted colorant and the mass of the colorant.

In another aspect, a liquid tinting system comprises a mixing system for mixing liquids, a color measurement unit for receiving and measuring a liquid sample collected from the mixing system to generate liquid color information, a color digitization processor coupled to the color measurement unit, a color digitization database storing product information and colorant information and coupled to the color digitization processor, and a memory storing computer-executable instructions. The instructions, when executed by the color digitization processor, configure the color tinting system for receiving, from the color measurement unit, the liquid color information, identifying a product identifier based on the liquid color information and the product information, and executing an artificial intelligence (AI) color prediction engine to predict colorants. Executing the Al color prediction engine comprises defining delta errors based on the liquid color information and the product identifier. Executing the Al color prediction engine further comprises modeling, by one or more models, tinting of the liquid sample by predicting one or more colorants and colorant masses based on the delta errors, the liquid color information, the product identifier, and the colorant information. The liquid tinting system further comprises transmitting the colorants and colorant masses to the mixing system.

In yet another aspect, a system for measuring liquid color comprises a testing container. The testing container comprises a base, an interior mount, and an interior compartment wherein the interior mount is coupled to the base. The system for measuring liquid further comprises a testing shelf wherein the testing shelf is mounted within the interior compartment. The testing shelf comprises a transparent interior surface configured to receive a test sample. The system for measuring liquid color further comprises a spectrophotometer coupled to the interior mount beneath the transparent interior surface of the testing shelf wherein the spectrophotometer is oriented such that a sensor end of the spectrophotometer is pointed towards the transparent interior surface.

Other objects and features of the present invention will be in part apparent and in part pointed out herein.

### BRIEF DESCRIPTION OF THE DRAWINGS

FIG. 1 illustrates an artificial intelligence (AI) based tinting assistant system according to an embodiment.
FIG. 2A illustrates a sample display generated by the insights processor showing tinting iterations required by product cluster where the clusters are created based on colors.
FIG. 2B illustrates an embodiment of software enabling selection of colorants to generate a prediction of the tinting weights of the colorants.
FIG. 3 illustrates a process for predicting colorants to tint using an artificial intelligence (AI) tinting prediction engine.
FIG. 4 illustrates an embodiment of the Al tinting prediction engine for use in the process of FIG. 3.
FIG. 5 illustrates a method for digitizing tinting process information and training an Al tinting prediction engine.
FIG. 6A illustrates an isometric view of a color measurement unit.
FIG. 6B illustrates the interior of the color measurement unit during measurement of a liquid batch.

Corresponding reference characters indicate corresponding parts throughout the drawings.

### DETAILED DESCRIPTION

The features and other details of the concepts, systems, and techniques sought to be protected herein will now be more particularly described. It will be understood that any specific embodiments described herein are shown by way of illustration and not as limitations of the disclosure and the concepts described herein. Features of the subject matter described herein can be employed in various embodiments without departing from the scope of the concepts sought to be protected.

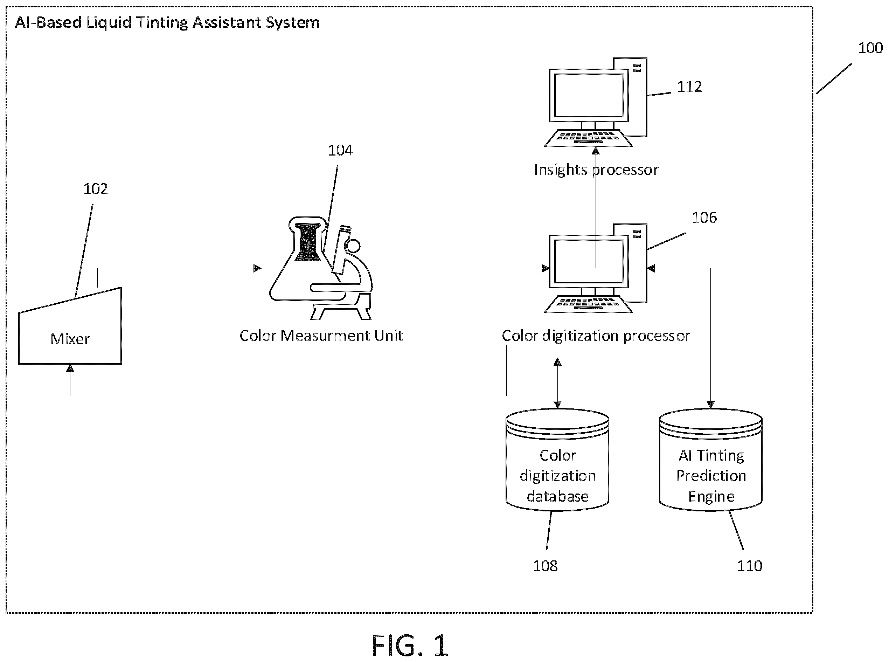

Referring to the figures and description below, a system 100 to tint liquids based on an artificial intelligence (Al), such as machine learning (ML), is disclosed. FIG. 1 is a block diagram illustrating the system 100 performing an example process embodying aspects of the disclosure. In one embodiment, an industrial mixing system 102 contains a liquid product to be tinted, such as a paint. In other embodiments, other liquid products may be tinted. In some embodiments the liquid product starts colored or tinted. A liquid batch sample is collected from the industrial mixing system 102 and transferred to a color measurement unit 104. The color measurement unit 104, further described below, measures the liquid batch sample to determine a color measurement value for the batch sample. As described above, a conventional tinting process requires using the liquid batch sample to create a batch test panel for comparison with a target reference panel rather than an evaluation of the liquid product itself. Aspects of the present disclosure increase the efficiency for production as it overcomes the need to repeatedly make test panels, thus, eliminating the time needed to create and cure multiple test panels. Further, the liquid color measurement system 100 reduces production costs for a batch because the system does not require the creation of physical batch test panels or product sample panels.

The color digitization processor 106 receives the liquid batch measurement and coordinates the tinting process. In some embodiments, the color digitization processor 106 couples with a display for displaying graphical information relating to the tinting process as well as input devices for configuring or updating the tinting process. In an embodiment, an operator of the tinting process may perform actions through interactive software, such as monitoring the tinting process, modifying aspects of the process reviewing the current tinting process, or reviewing historical tinting processes. The interactive software may be standalone or a component of the tinting process management software. The color digitization processor 106 connects with both a color digitization database 108 and an Al tinting prediction engine 110. In some embodiments, the Al tinting prediction engine 110 is executed by the color digitization processor 106.

The color digitization database 108 stores both information on the current tinting process, historical tinting processes, stock keeping unit (SKU) information for the target standard liquid samples and colorants added to tint the liquids. In some embodiments, the SKU information includes a SKU identifier, a SKU end application, color measurement values for the SKU, measurement dates, and an acceptable delta error. Historical batch tinting information can include information about the number of tinting operations, colorants required, colorant mass, colorant mixing times, batch sizes, batch dates, whether the batch was accepted and/or color measurement values for each product identifier, as well as information related to mapping colors to product identifiers. Colorant information stored within the color digitization database 108 includes one or more of colorant identifiers, and a strength measurement value.

In an embodiment, Al tinting prediction engine 110 comprises computer-executable instructions executed by the color digitization processor 106. The Al tinting prediction engine 110, as will be described in further detail below, receives the color measurement value, compares it to a corresponding value of a target color, predicts which colorants, and their masses, to be added to achieve the desired result matching the target color. The target color may be determined by a selected input color or automatically generated within the Al tinting prediction engine 110 based on the color values of the earlier accepted batches of the product.

The color digitization processor 106 transmits the prediction information to an insights processor 112. In some embodiments, the insights processor 112 operates as a component within the color digitization processor 106. In other embodiments, the insights processor 112 operates standalone connected by network. In some embodiments, the insights processor 112 is connected to the color digitization processor 106 through a wireless network such as Wi-Fi or Bluetooth. The insights processor 112 executes instructions for transmitting the prediction information to an external display.

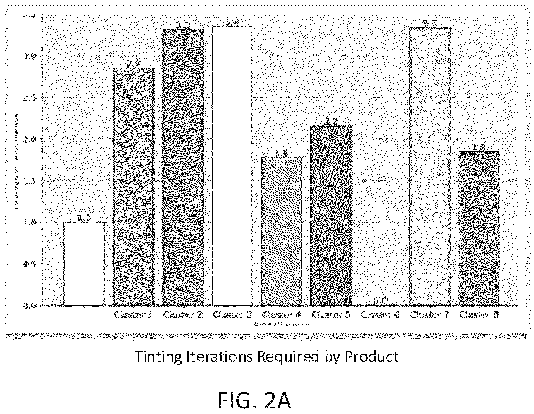

The insights processor 112 allows a plant manager or others supervising the tinting process access to details about the tinting process, including expected processing time and a predicted and/or actual number of iterations required to complete the tinting process. The insights processor 112 provides a plant manager or any other authorized plant personnel with real-time updates on the tinting process. Advantageously, up-to-date tinting process information enables an operator to accurately plan based on tinting process execution time thereby increasing plant efficiency. Further, the insights processor 112 in combination with the interactive software analyze the historical tinting information to display to an operator. The insight processor 112 evaluates the historical tinting information to evaluate products and provide feedback for the types of products requiring more iterations of tinting. For example, FIG. 2A illustrates a display showing tinting iterations required by product cluster according to an embodiment. The insights processor 112 and interactive software provide insights on the tinting process by clustering information from historical tinting processes to inform future tinting process planning.

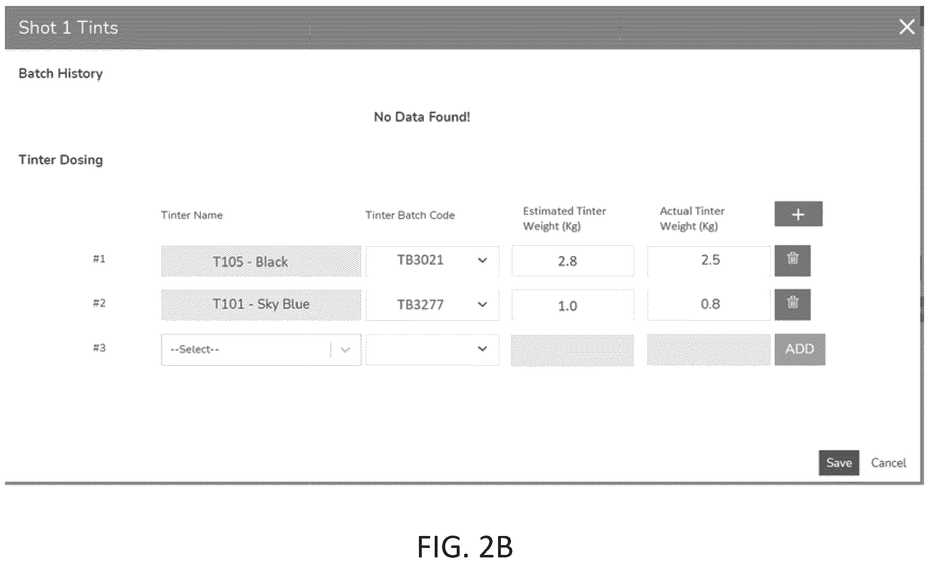

In some embodiments, the interactive software enables an operator to generate predictions for tinting process outcomes. As illustrated by FIG. 2B, in one embodiment, an operator inputs selected colorants and a target standard sample color to generate the prediction. Then the Al tinting prediction engine 110 predicts the mass of colorants to be added to achieve the desired target standard sample. Next, the operator performs tinting through the industrial mixing system 102 according to the prediction or the operator may override the prediction to alter the colorant masses. If the operator overrides the prediction, the changed values are transmitted to the color digitization database 108 to incorporate in retraining the Al tinting prediction engine 110. In another embodiment, a user inputs colorant data into the interactive software. Then, after selecting the colorants and mass of colorants to be used, the Al tinting prediction engine 110 generates a prediction of the delta effect on a liquid batch. The operator can adjust colorants added and the mass of colorants to find a desired outcome of the tinting process.

In parallel or serially with transmission to the insights processor 112, the color digitization processor 106 also transmits the predicted colorants and mass of colorants to the industrial mixing system 102 to complete the tinting process. The industrial mixing system 102 then adds the required colorants to the mixer to update the liquid batch. The resultant liquid batch may be processed multiple times to achieve the desired color by collecting subsequent measurement values.

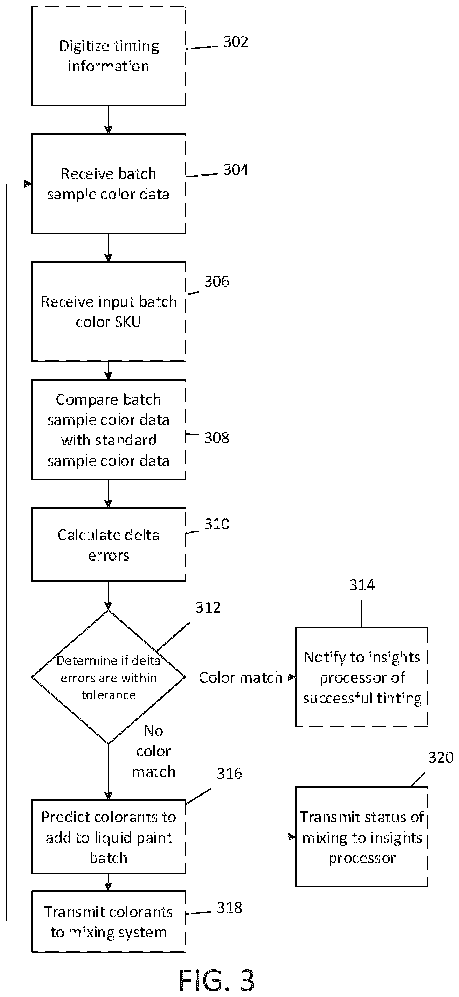

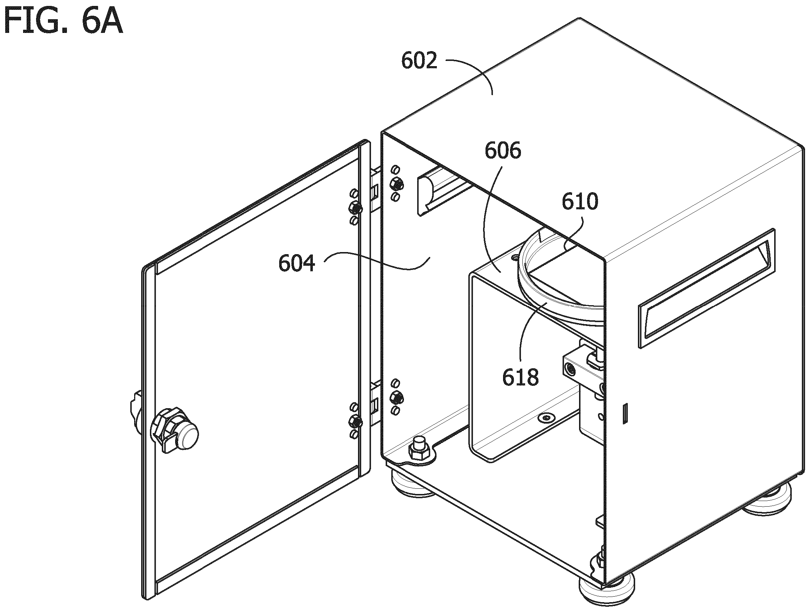

Referring now to FIG. 3, an example tinting process embodying aspects of the present disclosure is shown. The process begins with digitizing the tinting information at 302. Digitizing the batch information includes storing SKU liquid sample measurement values, colorant liquid sample measurement values, and previous batch sample measurement values in the color digitization database 108. The SKU liquid sample measurement values correspond to the potential target values for the tinting process. The process continues with receiving a measurement value of the batch sample color at 304. In some embodiments, the batch sample color is determined by a color measurement unit as seen in FIGS. 6A. In some embodiments, the color measurement value received by the color digitization processor 106 comprises a measurement in any of a variety of color spaces such as CIE XYZ, CIE L*a*b*, CIE L*C*h*, or CIELUV. After receiving the color measurement value, the operator inputs a product identifier or stock keeping unit (SKU) for the batch color at 306.

The tinting process continues at 308 by comparing the batch sample color measurement value with a standard reference measurement value for the target color. In some embodiments, comparing the batch sample color measurement value with the standard reference measurement value comprises calculating in the difference for each coordinate in a color space. For example, calculating the difference between each of the L*, a*, and b* coordinates in the CIELAB color space. In some embodiments, after calculating the difference for each coordinate, the color digitization processor 106 or Al tinting prediction engine 110 determines one or more delta error(s) at step 310. In one embodiment, a delta error in the CIELAB color space is determined by calculating the root mean square of all the coordinate differences. However, other methods of determining delta error(s) can be applied. The Al prediction engine 110 then evaluates a status based on the delta error(s) at step 312. In one embodiment, the Al prediction engine 110 predicts an acceptable tolerance of delta error(s) for the target standard sample value based on historical batch information including whether a given batch for the same SKU was accepted or rejected. If the delta error(s) are within an acceptable tolerance for the target standard sample value, the tinting process is complete. The color digitization processor 106 then transmits this status to the insights processor 112. In some embodiments, the color measurement unit captures another color measurement value for the resultant batch to update the color digitization database with further information.

Table I, below shows examples of batch information and calculating delta error within the CIELAB color space:

**TABLE I**

| | | | Liquid Sample Measurement | | | Standard Sample Measurement | | | Error | | | |
|---|---|---|---|---|---|---|---|---|---|---|---|---|
| Batch ID | SKU ID | Date | L | A | B | L | A | B | ΔL | ΔA | ΔB | ΔE |
| B1095 | S90H88 | 8/16/2024 | 83.72 | 0.72 | -1.07 | 84.02 | 0.71 | -0.86 | 0.30 | -0.01 | 0.21 | 0.37 |
| B1094 | S33FF6 | 8/16/2024 | 47.09 | 4.21 | -19.92 | 46.75 | 3.92 | -20.54 | -0.34 | -0.29 | -0.62 | 0.76 |
| B1093 | S79GD1 | 8/16/2024 | 78.82 | -0.55 | 8.75 | 78.77 | -0.47 | 8.75 | -0.05 | 0.08 | 0.00 | 0.09 |

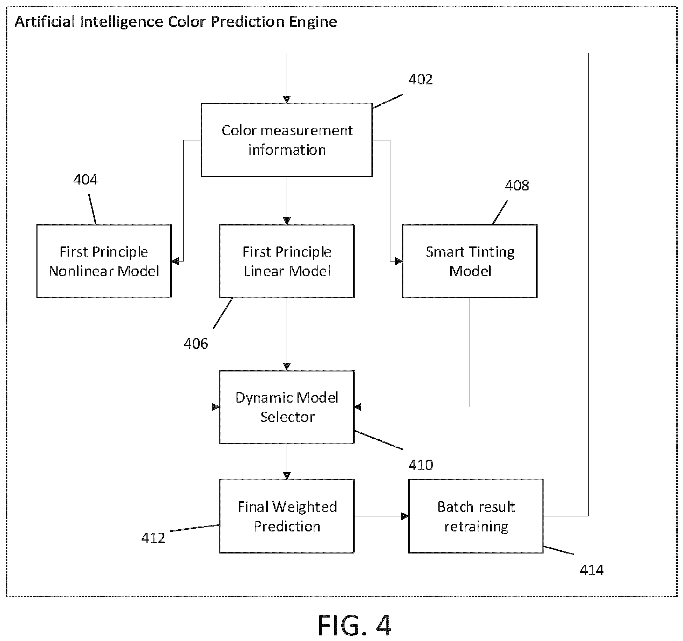

If the delta error(s) are not within an acceptable tolerance, the tinting process continues with further tinting. At step 316, the Al tinting prediction engine predicts colorants and mass of colorants needed to obtain the target standard sample measurement value by modeling the tinting process. This process is illustrated in FIG. 4, which is further described below. After predicting colorants to be added to the liquid batch, the color digitization processor 106 transmits the colorants and needed mass to the industrial mixing system 102 at step 318. In some embodiments, an operator may override the predicted mass of colorants. For example, an operator selects masses at 70% of the prediction in order to test at after the next iteration of mixing. The mixing system then proceeds with tinting the liquid batch with the colorants with the predicted mass or the operator input mass. After tinting the liquid, the color digitization processor 106 transmits the predicted colorants to the insights processor 112 along with information regarding expected tinting operations required and/or time to complete the tinting process at step 320. Then, after the mixing is completed by the industrial mixing system 102, the tinting may restart at step 304 by collecting a second batch sample from the resultant batch.

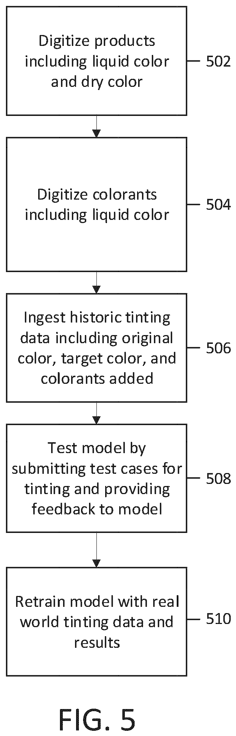

Referring now to FIG. 4, the Al tinting prediction engine comprises one or more models of the tinting process generating one or more predictions for tinting. The Al tinting prediction engine 110 begins by receiving the color measurement information 402. In one embodiment, color measurement information 402 comprises a color measurement value, such as a CIELAB coordinate, a calculation of delta error(s), a batch volume, and target color information which comprises a target color measurement value. Then one or more models weight the color information to predict which colorants and needed mass of each to add to the liquid batch to achieve a match to the target color after application and drying. FIG. 4 illustrates one embodiment where the Al prediction engine 110 feeds the color information into multiple models. Prediction generating models include any of various machine learning models. The present embodiment illustrates using one or more of a first principle nonlinear model 404, a first principle linear model 406, or a smart tinting model 408. The smart tinting model 408 is a machine learning model which has been trained based on historical tinting information and colorant information as described below, see FIG. 5.

Each model generates a prediction of weights for tinting. As each model receives the color measurement information 402, it generates a prediction. Each model may be separately trained and set different weights for each property under consideration depending on the model. In one embodiment, the models are weighted based on a strength factor. The measurement of color in the digital space may not accurately represent a color representation in the physical space. As such, one or more of the prediction models may be given a weighting to update colors after the initial prediction to better reflect physical space color. The prediction from each model is then fed into a dynamic model selector 410.

The dynamic model selector 410 generates a final weighted prediction 412 for the tinting operation. In some embodiments, the dynamic model selector 410 selects a single prediction of the input models based on historical tinting information and the target color. In some embodiments, the dynamic model selector 410 generates a composite weight based on the input models predictions. In still other embodiments, the dynamic model selector 410 acts as a second layer of Al prediction by generating weights for each model prediction to generate a single weight through a machine learning model.

After generation of the final weighted prediction 412, the batch result retrains the model 414. In some embodiments, the industrial mixing system 102 uses the final weighted prediction 412 to add colorants to a liquid batch. After, resultant generated color measurement information is fed back into the Al color prediction engine for retraining at 402. In testing the model, rather than feeding back in a new sample measured after the tinting process, the color measurement information 302 is a result from historical tinting data. Each of the models undergoes re-weighting of their various inputs including the dynamic model selector 412 in embodiments where the dynamic model selector is a machine learning model itself. Aspects to the present disclosure improve consistency of the tinting process. Conventional models of tinting require an expert in the tinting process to evaluate batch sample panels and standard reference panels to predict one or more colorants needed for the liquid batch. As a result, the process can vary based on the knowledge of the expert and subjective observation. However, the process as presently disclosed, improves the process by ensuring consistent predictions for a given target color due to the digitizing the knowledge of a tinting expert and consistent, standardized modeling through the Al tinting prediction engine 110.

Referring now to FIG. 5, the Al tinting prediction engine 110 comprises a tinting model trained on historical tinting information. First at step 502, products are digitized within the color digitization database 108. Digitization of products includes storing information comprising a product identifier or SKU, a color measurement value for the liquid product, a color measurement value for the dry product, and previous tinting information. Previous tinting information further comprises a color measurement value for the liquid product at each step of the tinting process, colorants added for each tinting step, mass of colorants added, and any dry color measurement values for each step available.

Next at step 504, colorant information is digitized for use within the Al tinting prediction engine 110. Digitizing colorant information includes storing information comprising a colorant identifier, any manufacturing information if available, and a colorant strength which can be measured, predicted, or provided by the manufacturer. A colorant strength represents the effectiveness of a colorant at tinting a liquid product. A colorant having a high colorant strength requires less mass of the colorant added to the liquid batch to achieve the desired outcome, whereas a colorant having a low colorant strength requires more mass of the colorant to achieve the desired outcome. Colorant information also comprises historical information regarding use of the colorant, such as which products include use of the colorant and mass of colorant used. In some embodiments, the Al tinting prediction engine 110 predicts a colorant strength by weighting previous historical tinting process information based on the mass of colorant used.

The training of the model begins at step 506 by ingesting the historical tinting information. A curated set of the historical tinting information is synthesized for training the model. The information is selected based on various criteria depending on factors such as tinting outcomes, reliability of the information, and sufficiency of detailed process information for each step of the tinting process. Similarly, the colorant information is also curated to optimize training of the model. As a part of curating the training set of information, a curated set of testing information will also be generated. Then the model is trained based off the curated historical tinting information as well as the curated colorant information.

After initial training of the model, the model is tested at step 508. Testing the model involves submitting curated historical tinting process information from the test set to the model to determine a prediction. The prediction is then compared to the historical process result to determine the accuracy of the prediction. Then the weights for the colorant information or product information are updated based on the accuracy of the prediction. Finally, the model retrains further at step 510 as the tinting process generates new tinting information to feed back into the model. As a result, the model continually learns from the generated predicting colorants and their resultant color measurement values.

Referring now to FIG. 6A, the system for tinting liquids in some embodiments implements color measurement unit 104. The color measurement unit 104 provides accurate color measurement of liquids including highly viscous liquids. The color measurement unit 104 in the illustrated embodiment comprises a container 602 within an interior area 604 in which to measure the liquid. The container 602 may be of any opaque material to prevent outside light from interfering with the measurement. In some embodiments, color measurement unit 104 further comprises a hinged door which seals to the exterior of the container 602 to prevent all light from interior area 604. The color measurement unit 104 further comprises an interior area 604 within container 602 designed for testing a liquid batch sample. Within the interior area 604, a testing shelf 606 is mounted inside container 602. In the present embodiment, the testing shelf 606 includes a transparent testing surface 610 formed therein. In some embodiments, the testing shelf 606 only comprises the transparent testing surface 610. The transparent testing surface 610 may be made of a light permeable material, such as glass or plastic, enabling the testing of the liquid batch sample from below the testing shelf 606.

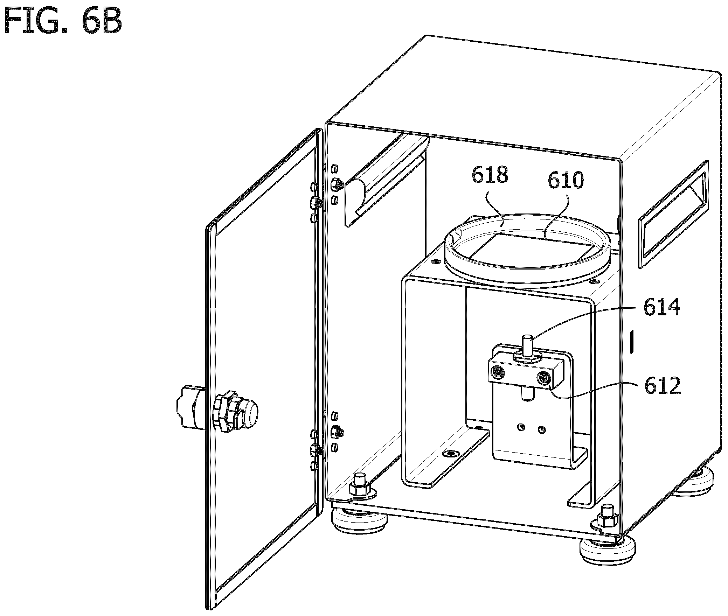

Referring now to FIG. 6B., beneath the testing shelf 606 coupled to the base of the container is a mount 612 for attaching a sensor 614. In some embodiments, the mount 612 is adjustable enabling the sensor 614 to be moved closer or further away from the transparent testing surface 610 for more accurate testing. In some embodiments the sensor 614 is a spectrophotometer. The spectrophotometer may be coupled with a controller or another device for transmitting the data captured by the spectrophotometer to the color digitization processor 106. An example of a suitable spectrophotometer is the Micro-Epsilon CFS2-M20-E-2400. Similarly, a suitable controller for transmitting the sensor data is the Micro-Epsilon colorSENSOR CFO200.

In some aspects, the position of the sensor 614 enables quick accurate testing of liquid samples. A batch sample within a testing container 618 is placed upon the transparent interior testing surface 610. Then the sensor 618 captures a color measurement value of the batch sample, which may be processed by a separate controller or the color digitization processor 106. As previously discussed, conventional methods of detecting color cause the spectrophotometer to be oriented above the test sample. According to the conventional arrangement, the meniscus of the liquid sample and/or air bubbles within a highly viscous liquid are causes of color measurement error. In the present embodiment, a sample can quickly be placed within the interior testing surface 610 and measured with the sensor 614 without significant reconfiguration to adjust for the meniscus of the liquid or delaying to allow air bubbles to escape the liquid. Moreover, color measurement unit 104 is capable of capturing color measurement values from a liquid sample utilizing the test configuration of FIGS. 6A and 6B thus avoiding the drying time associated with conventional color measurements.

In some embodiments, the color measurement unit 104 is coupled to the color digitization processor 106. The direct connection between the color measurement unit 104 and the color digitization processor 106 enables direct management of the sensor from the color digitization processor 106. Further, the direct link ensures safe rapid storage of the liquid batch measurement value without relying on an external network. In some embodiments, the color digitization processor 106 further comprises the insights processor 112 and the Al tinting prediction engine 110 as subcomponents of the tinting process software. In such an embodiment, the color digitization processor 106 operates the entirety of the tinting and prediction process and displays all information regarding the current tinting process and historical tinting processes. In some embodiments, the color measurement unit 104 and color digitization processor 106 couple by a wireless network, such as Wi-Fi or Bluetooth.

Embodiments of the present disclosure may comprise a special purpose computer including a variety of computer hardware, as described in greater detail herein.

For purposes of illustration, programs and other executable program components may be shown as discrete blocks. It is recognized, however, that such programs and components reside at various times in different storage components of a computing device, and are executed by a data processor(s) of the device.

Although described in connection with an example computing system environment, embodiments of the aspects of the invention are operational with other special purpose computing system environments or configurations. The computing system environment is not intended to suggest any limitation as to the scope of use or functionality of any aspect of the invention. Moreover, the computing system environment should not be interpreted as having any dependency or requirement relating to any one or combination of components illustrated in the example operating environment. Examples of computing systems, environments, and/or configurations that may be suitable for use with aspects of the invention include, but are not limited to, personal computers, server computers, hand-held or laptop devices, multiprocessor systems, microprocessor-based systems, set top boxes, programmable consumer electronics, mobile telephones, network PCs, minicomputers, mainframe computers, distributed computing environments that include any of the above systems or devices, and the like.

Embodiments of the aspects of the present disclosure may be described in the general context of data and/or processor-executable instructions, such as program modules, stored one or more tangible, non-transitory storage media and executed by one or more processors or other devices. Generally, program modules include, but are not limited to, routines, programs, objects, components, and data structures that perform particular tasks or implement particular abstract data types. Aspects of the present disclosure may also be practiced in distributed computing environments where tasks are performed by remote processing devices that are linked through a communications network. In a distributed computing environment, program modules may be located in both local and remote storage media including memory storage devices.

In operation, processors, computers and/or servers may execute the processor-executable instructions (e.g., software, firmware, and/or hardware) such as those illustrated herein to implement aspects of the invention.

Embodiments may be implemented with processor-executable instructions. The processor-executable instructions may be organized into one or more processor-executable components or modules on a tangible processor readable storage medium. Also, embodiments may be implemented with any number and organization of such components or modules. For example, aspects of the present disclosure are not limited to the specific processor-executable instructions or the specific components or modules illustrated in the figures and described herein. Other embodiments may include different processor-executable instructions or components having more or less functionality than illustrated and described herein.

The order of execution or performance of the operations in accordance with aspects of the present disclosure illustrated and described herein is not essential, unless otherwise specified. That is, the operations may be performed in any order, unless otherwise specified, and embodiments may include additional or fewer operations than those disclosed herein. For example, it is contemplated that executing or performing a particular operation before, contemporaneously with, or after another operation is within the scope of the invention.

When introducing elements of the invention or embodiments thereof, the articles "a," "an," "the," and "said" are intended to mean that there are one or more of the elements. The terms "comprising," "including," and "having" are intended to be inclusive and mean that there may be additional elements other than the listed elements.

Not all of the depicted components illustrated or described may be required. In addition, some implementations and embodiments may include additional components. Variations in the arrangement and type of the components may be made without departing from the spirit or scope of the claims as set forth herein. Additional, different or fewer components may be provided and components may be combined. Alternatively, or in addition, a component may be implemented by several components.

The above description illustrates embodiments by way of example and not by way of limitation. This description enables one skilled in the art to make and use aspects of the invention, and describes several embodiments, adaptations, variations, alternatives and uses of the aspects of the invention, including what is presently believed to be the best mode of carrying out the aspects of the invention. Additionally, it is to be understood that the aspects of the invention are not limited in its application to the details of construction and the arrangement of components set forth in the following description or illustrated in the drawings. The aspects of the invention are capable of other embodiments and of being practiced or carried out in various ways. Also, it will be understood that the phraseology and terminology used herein is for the purpose of description and should not be regarded as limiting.

It will be apparent that modifications and variations are possible without departing from the scope of the invention defined in the appended claims. As various changes could be made in the above constructions and methods without departing from the scope of the invention, it is intended that all matter contained in the above description and shown in the accompanying drawings shall be interpreted as illustrative and not in a limiting sense.

In view of the above, it will be seen that several advantages of the aspects of the invention are achieved and other advantageous results attained.

Accordingly, the present invention may be described as follows, optionally in combination with one or more of the above-mentioned features of the present invention: A method of tinting liquids in an industrial mixing system. A color measurement unit receives a liquid batch sample and measures the sample to generate a color measurement value. A color digitization processor receives the color measurement value and executes an artificial intelligence (AI) tinting prediction engine. The Al tinting prediction engine stores the color measurement value in a database, which contains a plurality of historical batch sample color information, a plurality of target standard sample color measurement values, and a plurality of colorant information. The Al tinting prediction engine then defines delta errors between the color measurement value and a target standard sample measurement value. The Al tinting prediction engine then generates predicted colorants and colorant masses based on the delta errors and information stored within the database. The predicted colorants are then transmitted to the mixing system and mixed into the liquid batch.

## Claims

1. Method of tinting liquids in an industrial mixing system, the method comprising:
collecting a liquid batch sample from a liquid batch in an industrial mixing system;
measuring the liquid batch sample with a sensor to generate a batch color measurement value associated with the liquid batch sample;
transmitting the batch color measurement value to a color digitization processor;
executing, by the color digitization processor, an artificial intelligence (AI) tinting prediction engine, wherein executing the Al tinting prediction engine comprises:
storing the batch color measurement value in a color digitization database, the color digitization database comprising a plurality of historical batch sample color information, a plurality of target standard sample color measurement values, and a plurality of colorant information wherein the colorant information includes a colorant value;
defining delta errors between the batch color measurement value and a target standard sample color measurement value of the target standard sample color measurement values; and
modeling tinting of the liquid batch by weighting one or more of the delta errors, the batch color measurement value, a batch volume, the plurality of historical batch sample color information, and the plurality of colorant information to generate one or more predicted colorants and masses thereof from the plurality of colorant information to achieve the target standard sample color measurement;
receiving, by the industrial mixing system, the predicted colorants and the masses thereof; and
mixing, by the industrial mixing system, one or more colorants into the liquid batch based on the predicted colorants and the masses thereof.

2. Method as set forth in claim 1, wherein executing the Al tinting prediction engine further comprises:
generating prediction information for tinting process completion for the liquid batch comprising an expected number of tinting operations and an expected time for tinting;
sending the prediction information for tinting completion to an insights processor wherein the insights processor is configured to transmit graphical data of the prediction for tinting to a display.

3. Method as set forth in claims 1 or 2, wherein the method further comprises:
collecting, after mixing the one or more colorants, a resultant liquid sample from the industrial mixing system;
analyzing the resultant liquid sample by the sensor to generate a resultant color measurement value associated with the resultant liquid sample;
transmitting the resultant color measurement value to the color digitization processor;
updating the color digitization database with the resultant color measurement value; and
re-weighting the plurality of historical batch sample color information and the plurality of colorant information based on the batch color measurement value, the predicted colorants, the mass of each of the predicted colorants, the target standard sample color measurement value, and the resultant color measurement value.

4. Method as set forth in any of claims 1 to 3, wherein the sensor comprises a spectrophotometer and the batch color measurement value comprises a CIELAB coordinate.

5. Method as set forth in any of claims 1 to 4, wherein executing the Al tinting prediction engine further comprises determining, before defining the delta errors, a target standard sample delta error tolerance based on the plurality of historical batch sample color measurement values and the target standard sample color measurement value.

6. Method as set forth in any of claims 1 to 5, wherein the colorant information further includes a colorant strength and wherein executing the Al tinting prediction engine further comprises:
generating, after storing the batch sample color, the colorant strength for each of the plurality of colorant information based on the plurality of historical batch sample color information and the plurality of colorant information.

7. Method as set forth in any of claims 1 to 6, wherein the method further comprises:
training a model, prior to executing the Al tinting prediction engine, wherein training the model comprises:
creating a plurality of curated standard color information, the curated standard color information comprising a product identifier, a standard liquid color, a standard dry color, and historical tinting data, to the artificial tinting prediction engine;
creating a plurality of curated colorant information, the curated colorant information comprising a colorant identifier, the historical tinting data, and a colorant liquid color, to the artificial tinting prediction engine;
determining a colorant strength for each of the curated colorant information;
training the model based off the curated standard color information, the curated colorant information, and the colorant strength for each of the curated colorant information by generating a plurality of weights;
generating one or more predicted test colorants based on a test liquid color;
regenerating the weights based on the predicted test colorants and one or more expected colorants.

8. Liquid tinting system comprising:
a mixing system for mixing liquids;
a color measurement unit for receiving and measuring a liquid sample collected from the mixing system to generate liquid color information;
a color digitization processor coupled to the color measurement unit;
a color digitization database coupled to the color digitization processor, the color
digitization database storing product information and colorant information; and a memory storing computer-executable instructions that when executed by the color
digitization processor, configure the color tinting system for:
receiving, from the color measurement unit, the liquid color information;
identifying a product identifier based on the liquid color information and the product information; and
executing an artificial intelligence (AI) color prediction engine to predict colorants, wherein executing the Al color prediction engine comprises:
defining delta errors based on the liquid color information and the product identifier;
modeling, by one or more models, tinting of the liquid sample by predicting one or more colorants and colorant masses based on the delta errors, the liquid color information, the product identifier, and the colorant information; and
transmitting the colorants and colorant masses to the mixing system.

9. Liquid tinting system as set forth in claim 8, wherein predicting the colorants and colorant masses is further based on a plurality of colorant strengths and wherein the color prediction processor is further configured for:
generating, after the identifying step, the plurality of colorant strengths for the colorant information.

10. Liquid tinting system as set forth in any of claims 8 or 9, wherein the system further comprises:
an insights processor, coupled by network to the color digitization processor, configured to transmit graphical information based upon the product information and colorant information to a display.

11. Liquid tinting system as set forth in any of claims 8 to 10, wherein the instructions in memory further comprise, after predicting one or more colorants and colorant masses:
estimating a number of iterations and an expected tinting time for tinting process completion;
transmitting the number of iterations, expected tinting time, and the colorants and colorant masses to the insights processor.

12. Liquid tinting system as set forth in any of claims 8 to 11, wherein executing the Al color prediction engine further comprises:
receiving an input representative of the colorants; and
predicting, before defining the delta errors, a resultant color based on the input representative of the colorants, the liquid color information, and the colorant information.

13. System for measuring liquid color, comprising:
a testing container comprising a base, an interior mount, and an interior compartment wherein the interior mount is coupled to the base;
a testing shelf wherein the testing shelf is mounted within the interior compartment and the testing shelf comprises a transparent interior surface configured to receive a test sample; and
a spectrophotometer coupled to the interior mount beneath the transparent interior surface of the testing shelf wherein the spectrophotometer is oriented such that a sensor end of the spectrophotometer is pointed towards the transparent interior surface.

14. System for measuring liquid color as set forth in claim 13, further comprising:
a color digitization processor connected by network to the spectrophotometer.

15. System for measuring liquid color as set forth in claims 13 or 14, wherein the interior mount comprises an adjustable mount, which enables the sensor to be moved closer to and away from the transparent interior surface.

## Amended claims

### Amended claims in accordance with Rule 137(2) EPC.

1. Method of tinting liquids in an industrial mixing system (102), the method comprising:
collecting a liquid batch sample from a liquid batch in an industrial mixing system (102);
measuring the liquid batch sample with a sensor (614) to generate a batch color measurement value associated with the liquid batch sample;
transmitting the batch color measurement value to a color digitization processor;
receiving, by the industrial mixing system (102), the predicted colorants and the masses thereof; and
mixing, by the industrial mixing system (102), one or more colorants into the liquid batch based on the predicted colorants and the masses thereof;
**characterized by** executing, by the color digitization processor (106), an artificial intelligence (Al) tinting prediction engine (110), wherein executing the Al tinting prediction engine comprises:
storing the batch color measurement value (302) in a color digitization database (108), the color digitization database (108) comprising a plurality of historical batch sample color information, a plurality of target standard sample color measurement values, and a plurality of colorant information wherein the colorant information includes a colorant value;
calculating delta errors between the batch color measurement value (302) and a target standard sample color measurement value of the target standard sample color measurement values; and
modeling tinting of the liquid batch by weighting one or more of the delta errors, the batch color measurement value (302), a batch volume, the plurality of historical batch sample color information, and the plurality of colorant information to generate one or more predicted colorants and masses thereof from the plurality of colorant information to achieve the target standard sample color measurement
wherein the method further comprises:
collecting, after mixing the one or more colorants, a resultant liquid sample from the industrial mixing system (102);
analyzing the resultant liquid sample by the sensor (614) to generate a resultant color measurement value (402) associated with the resultant liquid sample;
transmitting the resultant color measurement value (402) to the color digitization processor;
updating the color digitization database (108) with the resultant color measurement value (402); and
re-weighting the plurality of historical batch sample color information and the plurality of colorant information based on the batch color measurement value, the predicted colorants, the mass of each of the predicted colorants, the target standard sample color measurement value, and the resultant color measurement value (402).

2. Method as set forth in claim 1, wherein executing the Al tinting prediction engine further comprises:
generating prediction information for tinting process completion for the liquid batch comprising an expected number of tinting operations and an expected time for tinting;
sending the prediction information for tinting completion to an insights processor (112).

3. Method as set forth in claim 1 or 2, wherein the insights processor (112) is configured to transmit graphical data of the prediction for tinting to a display.

4. Method as set forth in any of claims 1 to 3, wherein the sensor (614) comprises a spectrophotometer and the batch color measurement value (302) comprises a CIELAB coordinate.

5. Method as set forth in any of claims 1 to 4, wherein executing the Al tinting prediction engine further comprises determining, before defining the delta errors, a target standard sample delta error tolerance based on the plurality of historical batch sample color measurement values and the target standard sample color measurement value.

6. Method as set forth in any of claims 1 to 5, wherein the colorant information further includes a colorant strength and wherein executing the Al tinting prediction engine (110) further comprises:
generating, after storing the batch sample color, the colorant strength for each of the plurality of colorant information based on the plurality of historical batch sample color information and the plurality of colorant information.

7. Method as set forth in any of claims 1 to 6, wherein the method further comprises:
training a model (414), prior to executing the Al tinting prediction engine (110), wherein training the model (414) comprises:
creating a plurality of curated standard color information, the curated standard color information comprising a product identifier, a standard liquid color, a standard dry color, and
historical tinting data, to the Al tinting prediction engine (110);
creating a plurality of curated colorant information, the curated colorant information comprising a colorant identifier, the historical tinting data, and a colorant liquid color, to the Al tinting prediction engine (110);
determining a colorant strength for each of the curated colorant information;
training the model based off the curated standard color information, the curated colorant information, and the colorant strength for each of the curated colorant information by generating a plurality of weights;
generating one or more predicted test colorants based on a test liquid color;
regenerating the weights based on the predicted test colorants and one or more expected colorants.

8. Liquid tinting system comprising:
a mixing system (102) for mixing liquids;
a color measurement unit (104) for receiving and measuring a liquid sample collected from the mixing system (102) to generate liquid color information;
a color digitization processor (106) coupled to the color measurement unit (104);
a color digitization database (108) coupled to the color digitization processor (106), the color digitization database (108) storing product information and colorant information; and
a memory storing computer-executable instructions that when executed by the color digitization processor (106), configure the liquid tinting system for:
receiving, from the color measurement unit (104), the liquid color information; and
identifying a product identifier based on the liquid color information and the product information;
**characterized in that** the memory storing computer-executable instructions that when executed by the color digitization processor (106), further configure the liquid tinting system for:
executing an artificial intelligence (AI) color prediction engine to predict colorants, wherein executing the Al color prediction engine comprises:
defining delta errors based on the liquid color information and the product identifier;
modeling, by one or more models, tinting of the liquid sample by predicting one or more colorants and colorant masses based on the delta errors, the liquid color information, the product identifier, and the colorant information; and
transmitting the colorants and colorant masses to the mixing system.

9. Liquid tinting system as set forth in claim 8, wherein predicting the colorants and colorant masses is further based on a plurality of colorant strengths and wherein the color prediction processor (106) is further configured for:
generating, after the identifying step, the plurality of colorant strengths for the colorant information.

10. Liquid tinting system as set forth in any of claims 8 or 9, wherein the system further comprises:
an insights processor (112), coupled by network to the color digitization processor (106), configured to transmit graphical information based upon the product information and colorant information to a display.

11. Liquid tinting system as set forth in any of claims 8 to 10, wherein the instructions in memory further comprise, after predicting one or more colorants and colorant masses:
estimating a number of iterations and an expected tinting time for tinting process completion;
transmitting the number of iterations, expected tinting time, and the colorants and colorant masses to the insights processor (112).

12. Liquid tinting system as set forth in any of claims 8 to 11, wherein executing the Al color prediction engine further comprises:
receiving an input representative of the colorants; and
predicting, before defining the delta errors, a resultant color based on the input representative of the colorants, the liquid color information, and the colorant information.

13. System for measuring liquid color, comprising:
a liquid tinting system as set forth in any of claims 8 to 12;
a testing container (618) comprising a base, an interior mount (612), and an interior compartment wherein the interior mount (612) is coupled to the base;
a testing shelf (606) wherein the testing shelf (606) is mounted within the interior compartment and the testing shelf (606) comprises a transparent interior surface (610) configured to receive a test sample; and
a spectrophotometer coupled to the interior mount (612) beneath the transparent interior surface (610) of the testing shelf (606) wherein the spectrophotometer is oriented such that a sensor end of the spectrophotometer is pointed towards the transparent interior surface (610).

14. System for measuring liquid color as set forth in claim 13, further comprising:
a color digitization processor connected by network to the spectrophotometer.

15. System for measuring liquid color as set forth in claims 13 or 14, wherein the interior mount (612) comprises an adjustable mount, which enables the sensor (614) to be moved closer to and away from the transparent interior surface (610).
